(19) Europäisches Patentamt
European Patent Office
Office européen des brevets

(11) **EP 2 647 989 B1**

(12) **EUROPÄISCHE PATENTSCHRIFT**

(45) Veröffentlichungstag und Bekanntmachung des Hinweises auf die Patenterteilung:
**03.06.2015 Patentblatt 2015/23**

(51) Int Cl.:
***G01N 27/72*** *(2006.01)*

(21) Anmeldenummer: **13158491.4**

(22) Anmeldetag: **11.03.2013**

(54) **Verfahren zur zerstörungsfreien quantitativen Bestimmung der Mikroeigenspannung II. und/oder III. Art**

Method for non-destructive quantitative determination of the micro-tension II and/or III Type

Procédé de détermination quantitative non destructive de la microcontrainte résiduelle de type II et/ou III.

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.04.2012 DE 102012007062**

(43) Veröffentlichungstag der Anmeldung:
**09.10.2013 Patentblatt 2013/41**

(73) Patentinhaber:
• **FRAUNHOFER-GESELLSCHAFT zur Förderung der angewandten Forschung e.V.**
**80636 Munich (DE)**
• **Universität Stuttgart**
**70174 Stuttgart (DE)**

(72) Erfinder:
• **Dr.-Ing. Altpeter, Iris**
**66123 Saarbrücken (DE)**
• **Dr.-Ing. Rabung, Madalina**
**66119 Saarbrücken (DE)**
• **Dr.-Ing. Szielasko, Klaus**
**66125 Dudweiler (DE)**

• **Prof.Dr. Schmauder, Siegfried**
**73252 Lenningen (DE)**
• **Dr. Binkele, Peter**
**71134 Aidlingen (DE)**

(74) Vertreter: **Pfenning, Meinig & Partner GbR**
**Patent- und Rechtsanwälte**
**Theresienhöhe 11a**
**80339 München (DE)**

(56) Entgegenhaltungen:
• **ALTPETER I ET AL: "Micro-magnetic evaluation of micro residual stresses of the IInd and IIIrd order", NDT & E INTERNATIONAL, BUTTERWORTH-HEINEMANN, OXFORD, GB, Bd. 42, Nr. 4, 1. Juni 2009 (2009-06-01), Seiten 283-290, XP026064959, ISSN: 0963-8695, DOI: 10.1016/J.NDTEINT.2008.11.007 [gefunden am 2009-04-03]**
• **Madalina Pîrlog ET AL: "Quantitative Non-Destructive Detection of Residual Stresses of the 2 nd and 3 rd Order by Using Micro Magnetic Methods", , 29. September 2006 (2006-09-29), XP055115332, Gefunden im Internet: URL:http://www.ndt.net/article/ecndt2006/doc/Fr.2.4.1.pdf [gefunden am 2014-04-25]**

**Beschreibung**

Die vorliegende Erfindung betrifft Verfahren zur zerstörungsfreien quantitativen Bestimmung der Mikroeigenspannungen II. und/oder III. Art, die auf Differenzbildung der maximalen Werte der Lastspannungsabhängigkeit der maximalen Barkhausenrauschamplituden an einem Prüfkörper vor und nach Auslagerung des Prüfkörpers in bestimmte thermische Auslagerungszustände beruht. Die vorliegende Erfindung ermöglicht somit die unabhängige Bestimmung der Mikroeigenspannung II. oder III. Art die gleichzeitige und aufgelöste Bestimmung der Mikroeigenspannungen II. und III. Art, sowie die Bestimmung der Summe der beiden Arten von Mikroeigenspannungen.

Nanoskalige, kohärente Ausscheidungen spielen bei der Werkstoffverfestigung eine dominante Rolle. Die Werkstoffverfestigung beruht auf den Mechanismen der Ausscheidungshartung, welche auf der Behinderung der Versetzungen durch fein verteilte kohärente Ausscheidungen sekundärer Phasen basiert. Es wurde nachgewiesen, dass im Falle des Fe-Cu-Systems die kohärenten Cu-Ausscheidungen (kleiner als 4 nm Durchmesser) von Versetzungen geschnitten werden, da es aufgrund der mechanisch-elastischen Eigenschaften für einen Teil einer Versetzungslinie energetisch günstiger ist, sich innerhalb der Ausscheidung zu befinden als außerhalb. Diese kohärenten Ausscheidungen sind von Mikroeigenspannungen umgeben, die unter anderem ein Maß für die Existenz solcher Ausscheidungen sind. Zurzeit gibt es für die Systeme Fe-Cu und Fe-Cu-(Ni-Mn) weltweit keine zerstörungsfreie Messmethode zur Ermittlung von Mikroeigenspannungen II. und III. Art.

Unter Eigenspannungen versteht man im werkstoffwissenschaftlichen Zusammenhang das Vorhandensein von Spannungen im Inneren eines Festkörpers, ohne dass äußere Kräfte oder Momente auf ihn einwirken.

Grundsätzlich werden Eigenspannungen in Makro- und Mikroeigenspannungen unterteilt. Die Makroeigenspannungen, auch Eigenspannungen I. Art genannt, zeichnen sich dadurch aus, dass sie über eine größere Anzahl von Kristalliten nahezu homogen sind. Die Mikroeigenspannungen werden unterteilt in die Eigenspannungen II. Art, die in mikroskopischen Bereichen homogen sind und die Eigenspannungen III. Art, die schon über wenige Atomabstände veränderlich sind. Im Realfall ist mit einer Überlagerung von Eigenspannungen I., II. und III. Art zu rechnen. Das Entstehen von Eigenspannungen kann verschiedene Ursachen haben. Beim Abkühlen bilden sich z.B. als Folge von Temperaturgradienten zwischen den äußeren und inneren Werkstoffbereichen thermische Eigenspannungen I. Art aus. Der Rand kühlt zunächst schneller ab als der Kern, was zur Bildung von Zugspannungen im Randbereich und Druckspannungen im Kern führt. Ist die Abkühlung hinreichend schnell, kann der Betrag der Zugspannungen so groß werden, dass sie im Randbereich durch plastische Deformation teilweise abgebaut werden. Die Druckspannungen im Kern bleiben aber aufgrund der Verfestigung im Randbereich weitestgehend erhalten. Mit Ausgleich des Temperaturgradienten kommt es daher zu einer Spannungsumkehr. Nach dem vollständigen Abkühlen bleiben im Randbereich also Druck- und im Kern Zugspannungen erhalten.

In Werkstoffen mit heterogenem Gefügeaufbau entstehen aufgrund der unterschiedlichen Wärmeausdehnungskoeffizienten der einzelnen Gefügebestandteile thermisch induzierte Eigenspannungen II. Art. Sie entstehen auch bei sehr langsamen Abkühlgeschwindigkeiten. Eine theoretische Abschätzung für den zweiphasigen Werkstoff ist folgendermaßen möglich:

$$\sigma_{\text{therm. II}} = \rho \cdot (\alpha_1 - \alpha_2) \cdot \Delta T$$

(mit thermisch induzierter Eigenspannung II. Art $\sigma_{\text{therm, II}}$, werkstoffspezifischer Konstante p, thermischen Ausdehnungskoeffizfenten $\alpha_i$ und Temperaturdifferenz $\Delta T$)

Die Änderung der Gitterparameter durch die Entspannung der Matrix bei der Ausscheidung einer zweiten Phase führt zu einer Volumenänderung in der Matrix. Das von der zweiten Phase in Anspruch genommene Volumen ist in der Regel ungleich der Volumenänderung der Matrix was zur Entstehung von so genannten Ausscheidungseigenspannungen führt. Im Falle von kohärenten Ausscheidungen entstehen durch die elastischen Gitterverzerrungen an den Grenzflachen zwischen Ausscheidungen und Matrix zusätzlich noch Kohärenzeigenspannungen III. Art.

Abhängig von der Art, wie sich eine äußere Belastung und der Eigenspannungszustand überlagern, kann das mechanische Verhalten von Bauteilen sowohl positiv als auch negativ beeinflusst werden. Die Dauerfestigkeit von Bauteilen kann z.B. durch das Einbringen von Druckeigenspannungen in den Randbereich (z.B. durch Sandstrahlen) positiv beeinflusst werden. Andererseits können z.B. thermisch induzierte Eigenspannungen in Schweißnähten zu frühzeitigem Bauteilversagen führen. Die Abschätzung des Einflusses des Eigenspannungszustandes ist im Allgemeinen sehr schwierig, da seine Charakterisierung im Realfall sehr kompliziert ist.

Speziell bei einer Überlagerung mit äußeren Lastspannungen kann es zur Ausbildung von mehrachsigen Spannungszuständen und damit verbundenen negativen Effekten kommen. Als Schlagwort wäre hier zum Beispiel die so genannte Spannungsrisskorrosion zu nennen. Die Eigenspannungen in einem Bauteil können üblicherweise durch eine geeignete Wärmebehandlung stark verringert werden.

Referenzverfahren zur quantitativen Ermittlung von Mikroeigenspannungen sind die Transmissionselektronenmikroskopie, die Röntgen-Diffraktometrie und Neutronen-Diffraktometrie. Diese Verfahren sind jedoch sehr zeitaufwändig, teuer und stellen besondere Anforderungen an die Probengeometrie, was ihren wirtschaftlichen Einsatz stark einschränkt.

I. Altpeter et al. beschreiben in "Micro-magnetic evaluation of micro residual stresses of the IInd and IIIrd order", NDT&E International 42 (2009), 283-290, Verfahren zur Bestimmung von Mikroeigenspannungen der II. oder der III. Art. Bei dem dort beschriebenen Verfahrensansatz konnten allerdings die Mikroeigenspannungen II. und III. Art nicht voneinander getrennt werden.

Derzeit gibt es für Fe- und Cu-haltigen Legierungssysteme, insbesondere Fe-Cu und Fe-Cu-(-(Ni-Mn), keine zerstörungsfreie Messmethode zur Ermittlung von Eigenspannungen II. und III. Art, insbesondere kein Messverfahren, das sowohl die quantitative Bestimmung der Mikroeigenspannung II. und gleichzeitig III. Art ermöglicht. Aufgabe der vorliegenden Erfindung ist es daher, Messverfahren anzugeben, mit denen sich auf einfache Art und Weise quantitativ die Mikrospannungen II. und III. Art in eisen- und kupferhaltigen Legierungssystemen bestimmen lassen.

Diese Aufgabe wird mit einem Verfahren zur zerstörungsfreien quantitativen Bestimmung der Mikroeigenspannung II. und gleichzeitig III. Art (thermisch induzierte Eigenspannung II. Art) mit den Merkmalen des Patentanspruches 6 gelöst. Das Verfahren ermöglicht zudem die Bestimmung der Summe der beiden Arten von Mikroeigenspannungen. Patentanspruch 1 gibt ein Verfahren zur zerstörungsfreien quantitativen Bestimmung der Mikroeigenspannung II. Art an, während Patentanspruch 5 ein Verfahren zur quantitativen Bestimmung der Mikroeigenspannung III. Art beschreibt. Die abhängigen Patentansprüche stellen vorteilhafte Weiterbildungen dar.

In einer ersten Ausführungsform betrifft die vorliegende Erfindung ein Verfahren zur zerstörungsfreien quantitativen Bestimmung der Mikroeigenspannung III. Art (Kohärenz-Zugeigenspannung III. Art, $\sigma_{Koh.\ III}$) und II. Art (thermisch induzierte Eigenspannung II. Art, $\sigma_{therm.\ II}$) und/oder der Summe der Mikroeigenspannungen II. und III. Art ($\Delta\sigma = \sigma_{therm.\ II} + \sigma_{Koh.\ III}$) eines Fe- und Cu-haltigen Legierungssystems mit einem Cu-Gehalt von $\leq 2$ Gew.-% umfassend, folgende Schritte:

a) Bestimmung des Lastspannungswertes $\sigma_1$, bei dem die Lastspannungsabhängigkeit der maximalen Barkhausenrauschamplitude $M_{MAX}(\sigma)$ für einen Prüfkörper aus dem Legierungssystem in einem Ausgangszustand (d.h. einem Zustand vor thermischer Auslagerung), ein Maximum aufweist,

b) erste Auslagerung des Prüfkörpers bis zur ausschließlichen Bildung von kohärenten Cu-Ausscheidungen,

c) Bestimmung des Lastspannungswertes $\sigma_2$, bei dem die Lastspannungsabhängigkeit der maximalen Barkhausenrauschamplitude $M_{MAX}(\sigma)$ für einen Prüfkörper Prüfkörpers nach der ersten Auslagerung und Abkühlung ein Maximum aufweist

d) weitere Auslagerung des Prüfkörpers bis zur Ostwald-Reifung,

e) Bestimmung des Lastspannungswertes $\sigma_3$, bei dem die Lastspannungsabhängigkeit der maximalen Barkhausenrauschamplitude $M_{MAX}(\sigma)$ für einen Prüfkörper nach der weiteren Auslagerung bis zur Ostwald-Reifung und Abkühlung ein Maximum aufweist,

f) Ermittlung der Mikroeigenspannung II. Art ($\sigma_{therm.\ II}$) als Differenz $\sigma_{therm.\ II} = |\sigma_1 - \sigma_3|$

g) Ermittlung der Mikroeigenspannung III. Art durch

i. Bilden der Summe der Mikroeigenspannung II. Art und der Mikroeigenspannung III. Art als Differenz

$$\Delta\sigma = \sigma_{therm.\ II} + \sigma_{Koh.\ III} = \left|\sigma_1 - \sigma_2\right|$$

und anschließender Differenzbildung

$$\sigma_{Koh.\ III} = \left|\Delta\sigma - \sigma_{therm.\ II}\right|$$

oder

ii. als Differenz

$$\sigma_{Koh. III} = |\sigma_3 - \sigma_2|.$$

Das erfindungsgemäße Verfahren beruht auf der Erkenntnis, dass während der ersten Auslagerung des Prüfkörpers bis zur ausschließlichen Bildung kohärenten Cu-Ausscheidungen sich Mikroeigenspannung II. Art aufaut, während bei der sich anschließenden Auslagerung des Prüfkörpers bis zur Ostwald-Reifung sich Mikroeigenspannung III. Art abbaut. Das Maximum der Barkhausenrauschamplitude $M_{MAX}$ ist von der externen Lastspannung abhängig. Diese Lastspannungsabhängigkeit der maximalen Barkhausenrauschamplitude, die sog. $M_{Max}(\sigma)$-Kurve, weist ein Maximum auf. Durch Vergleich der Lage des Maximums der $M_{MAX}(\sigma)$-Kurve vor bzw. nach bestimmten thermischen Auslagerungszuständen kann auf die Mikroeigenspannung II. und/oder III. Art geschlossen werden.

Die erfindungsgemäß verwendeten Begriffe und Terminologien werden dabei wie folgt verstanden:

<u>Maximum der Barkhausenrauschamplitude:</u>

Beim Ummagnetisieren behindern Gitterinhomogenitäten die Bloch-Wandbewegungen, als Folge kommt es während der eigentlich reversiblen Verschiebung der Bloch-wände immer wieder zum Stoppen der Bewegung an energetischen Hindernissen, die erst bei einer Erhöhung des äußeren Feldes und somit der Triebkraft des Ummagnetisierungsprozesses sprungartig, durch so genannte Barkhausensprüngen der Bloch-Wände, überwunden werden. Diese Sprungprozesse sind irreversibel. d.h. sie sind der Grund der Energiedissipation während der Magnetisierung.

Mit einem Barkhausensprung geht eine schlagartige Ummagnetisierung eines kleinen Werkstoffbereichs einher. Dies bewirkt eine schnelle Flussdichteänderung des Magnetfeldes, die ihrerseits Wirbelströme im umgebenden Material induziert. Die Wirbelströme führen wiederum zu Flussdichteänderungen in den sie umgebenden Volumenbereichen.

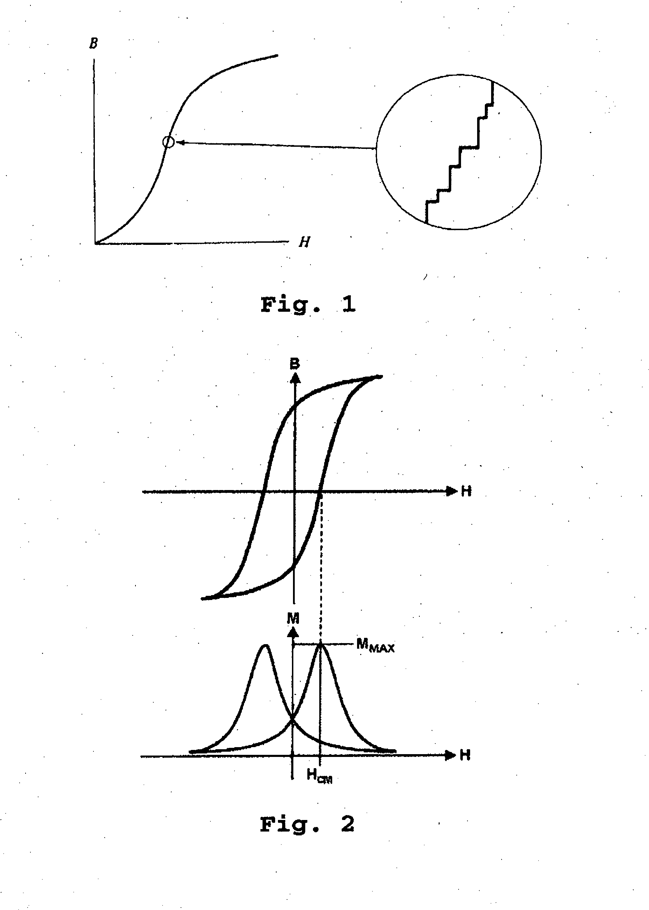

Die Wirbelströme diffundieren zur Werkstoffoberfläche, wo sie mit Hilfe einer Spule als Spannungspulse erfasst werden können. Da sich diese Spannungspulse in einem Lautsprecher wie Prasseln oder Rauschen anhören, spricht man von Barkhausenrauschen. Hierbei handelt es sich um einen stochastischen Prozess. Der makroskopisch gemessene Magnetisierungsvorgang einer Probe setzt sich aus vielen keinen Sprüngen zusammen, so dass die Hysterese aus vielen kleinen Stufen (vgl. Figur 1) besteht. Im steilsten Bereich der Hysterese, in der Nähe der Koerzitivfeldstärke $H_C$ dominieren 180°-Wandverschiebungsprozesse den Ummagnetisierungsvorgang. Deshalb sind bei der Durchsteuerung dieses Bereichs auch die meisten Sprungprozesse zu beobachten. Figur 2 zeigt eine Barkhausenrauschprofilkurve. Hierbei wird die aus den Sensorsignalen abgeleitete "Rauschaktivität" M als Funktion der magnetischen Tangentialfeldstärke H dargestellt. Wie erwartet, hat die Barkhausenrauschprofilkurve ein Maximum bei einem Feldstärkewert in der Nähe der Koerzitivfeldstärke $H_C$.

Experimentell kann die Barkhausenrauschprofilkurve folgendermaßen gewonnen werden:

1. Messung des zeitlichen Verlaufs der magnetischen Tangentialfeldstärke H und der Flussdichteänderung $\frac{d\phi}{dt}$, dabei wird stets die in gleicher Richtung der Flussdichteänderung gemessen, d.h. entweder bei ansteigender oder abnehmender Flussdichte

2. Hochpassfilterung des Zeitsignals der Flussdichteänderung um die stochastische Signalanteile der Barkhausensprünge vom Restsignal zu trennen.

3. Gleichrichtung des gefilterten Signals mit anschließender Tiefpassfilterung zur Bildung der Einhüllenden. Das resultierende Signal wird als M bezeichnet.

4. Zur Bildung der Barkhausenrauschprofilkurve wird das gleichgerichtete Signal M über dem gemessenen Zeitsignal der Tagentialfeldstärke H aufgetragen. Diese Profilkurve weist ein Maximum auf, $M_{MAX}$ bezeichnet.

Eine detaillierte Verfahrensvorschrift zur Bestimmung des Maximums der Barkhausenrauschamplitude, das auch im Rahmen der vorliegenden Anmeldung angewandt wird, ist bei I. Altpeter et al., NDT&E International 42 (2009), 283-290, beschrieben. Bezüglich des Verfahrens zur Bestimmung des Maximums der Barkhausenrauschamplitude sei insofern auf den zuvor zitierten Artikel verwiesen.

Ostwald-Reifung:

Bei der Ostwald-Reifung von ausscheidungsfähigen Legierungssystemen vergrößert sich der mittlere Teilchendurchmesser und der Teilchenabstand bereits ausgeschiedener kristalliner Domänen, während der ausgeschiedene Volumenanteil dieser Domänen konstant bleibt. Dies wird dadurch möglich, dass kleinere Teilchen von größeren aufgezehrt werden, so dass sich die größeren Teilchen vergröbern. Da die Teilchen selbst nicht wandern können, werden diese Vorgänge durch atomare Diffusion dominiert.

Härteverlauf:

In jedem zeitlichen Härteverlauf von ausscheidungsfähigen Legierungen spiegelt sich die Umwandlung von kohärenten über teilkohärente in inkohärente Ausscheidungen während der thermischen Auslagerung wider. Ein typischer Härteverlauf zeichnet sich durch einen ansteigenden Ast aus, in welchem eine Legierung mit hauptsächlich kohärenten Ausscheidungen vorliegt, ein Härtemaximum, in dem teilkohärente Ausscheidungen überwiegen und einen abnehmenden Ast, in dem inkohärente Ausscheidungen vorliegen und in dem Ostwald-Reifungsprozesse stattfinden.

Auslagerung:

Erfindungsgemäß wird unter dem Begriff "Auslgerung" eine thermisch Auslagerung verstanden. Bei einer thermischen Auslagerung wird eine Wärmebehandlung zur Erhöhung der Härte und Festigkeit des Prüfkörpers durchgeführt. Bei der Auslagerung findet die Ausscheidung von metastabilen Phasen in feinverteilter Form statt, so dass diese ein wirksames Hindernis für Versetzungsbewegungen des Prüfkörpers darstellen, WOdurch die Zunahme der Härte erklärt wird. Bei der Auslagerung findet somit eine Ausscheidungshärtung statt.

In dem oben genannten Schritten a), c) und/oder e) des erfindungsgemäßen Verfahrens erfolgt somit eine Aufnahme der Barkhausenrauschamplitudenkurven sowie die Bestimmung der Maxima der Lastspannungsabhängigkeit ($M_{MAX}(\sigma)$), wobei jeweils die angelegte mechanische Lastspannung an den Prüfkörper gegen den erhaltenen maximalen Wert der Barkhausenrauschamplitude $M_{MAX}$ aufgetragen wird. An dem Prüfkörper wird somit eine mechanische Lastspannung angelegt, konstant gehalten und der maximale Wert der Barkausenrauschamplitude bestimmt. Diese Messung wird bei verschiedenen externen mechanischen Lastspannungen wiederholt. Eine mechanische Lastspannung kann dabei eine Zuglastspannung aber auch eine Drucklastspannung sein, die mit externen mechanischen Mitteln an den Prüfkörper angelegt wird. Nimmt man das Maximum der Barkhausenrauschprofilkurve $M_{MAX}$ als Funktion einer Angelegten Lastspannung $\sigma$ auf, ergibt sich typischerweise die sog. $M_{MAX}(\sigma)$-Kurve. Diese Kurve durchläuft wiederum ein Maximum, das die maximale Lastspannungsabhängigkeit des maximalen Wertes der Barkhausenrauschamplitude repräsentiert. Die Mikroeigenspannungen II. bzw. III. Art kann jeweils als Betrag der Differenzspannung zwischen zwei Probezuständen angeben werden.

Beim oben angegebenen erfindungsgemäßen Verfahren erfolgt in einem ersten Schritt die Ermittlung des maximalen Wertes der Barkhausenrauschamplitude $M_{MAX}$ in Abhängigkeit einer variablen externen Lastspannung $\sigma$ eines Prüfkörpers (die sog. $M_{MAX}(\sigma)$-Kurve), der aus dem Legierungssystem gebildet ist. Der Prüfkörper liegt dabei in einem Ausgangszustand vor. Als Ausgangszustand kann dabei ein lösungsgeglühter und abgeschreckter, jedoch noch nicht thermisch ausgelagerter, Körper eingesetzt werden. Nach Ermitteln des Maximums der sog. $M_{MAX}(\sigma)$-Kurve ergibt sich eine Zuglastspannung $\sigma_1$, die mit dem Maximum der Barkhausenrauschamplitude korreliert.

Anschließend erfolgt eine erste Auslagerung des Prüfkörpers bis zu ausschließlichen Bildung kohärenten Cu-Ausscheidungen. Maßgeblich bei diesem Schritt ist, dass sich noch keine oder lediglich im untergeordneten Maße teil- und/oder inkohärente Cu-Ausscheidungen gebildet haben. Die Bildung von kohärenten Ausscheidungen kann anhand der Prüfung der Härte des Prüfkörpers während dieses Auslagerungsschrittes verfolgt werden. Solange die Härte bei der vorgenannten thermischen Auslagerung noch zunimmt, liegen keine bzw. lediglich im untergeordneten Maße teil- und/oder inkohärente Cu-Ausscheidungen vor. Der erste Auslagerungsschritt sollte daher bevorzugt derart ausgeführt werden, dass eine Zunahme der Härte des Prüfkörpers erzielt wird, jedoch das bei der thermischen Auslagerung erreichbare theoretische Härtemaximum nicht erreicht wird. Das Erreichen dieses Zustandes kann anhand einfacher Experimente (z.B. Ermittlung der Vickers-Härte an Härtetestproben) ermittelt werden.

Das Härtemaximum kann aus dem Verlauf der ermittelten Vickershärte als Funktion der Auslagerungsdauer bestimmt werden. Der Härtewert, bei dem hauptsächlich kohärente Cu-Ausscheidungen in der Fe-Matrix vorliegen, kann z.B. um 10 HV5 kleiner als das Härtemaximum gewählt werden.

Die Vorgehensweise bei der ersten Auslagerung basiert dabei auf den Erkenntnissen von Ogi et a1. ("Snoek relaxation in a Copper-precipitated alloy steel", Journal of Alloys and Compounds, 310 (2000), 432 bis 435), wonach bei thermischer Alterung von Cu-Fe-Legierungen eine Härtezunahme zu beobachten ist.

Beispielhafte, im Rahmen der vorliegenden Erfindung realisierte Auslagerungsdauern bei 500 °C zur Erzielung des ersten Auslagerungszustandes sind in der nachfolgenden beispielhaften Tabelle angegeben. Die verwendeten Proben dabei waren binäre Eisen-Kupfer-Legierungen, wobei sich mit zunehmenden Kupfergehalt kürze Auslagerungsdauern ergeben. Bei den angegebenen Auslagerungsdauern wurden lediglich kohärente Kuper-Ausscheidungszustände erhalten.

Tabelle 1: Auslagerungsdauer zur Einstellung kleiner, kohärenter Cu-Ausscheidungszustände

| Gew.% Cu | 0,5 | 0,65 | 0,75 | 0,9 | 1,0 | 1,2 | 1,4 | 1,5 | 1,7 | 1,9 |
|---|---|---|---|---|---|---|---|---|---|---|
| Auslagerungsdauer bei 500°C [h] | 35 | 14 | 10 | 7,83 | 6,5 | 4,5 | 3,5 | 3 | 1,5 | 1,17 |

Der derart ausgelagerte Prüfkörper wird erneut einer Bestimmung der $M_{MAX}(\sigma)$-Kurve unterzogen. Diese Bestimmung erfolgt in analoger Weise wie die Bestimmung der $M_{MAX}(\sigma)$-Kurve im ersten Schritt. Es wird eine zweite Lastspannung $\sigma_2$ erhalten, bei der die nunmehr aufgenommene der $M_{MAX}(\sigma)$-Kurve ein Maximum aufweist.

Anschließend erfolgt eine weitere Auslagerung des Prüfkörpers bis zur Ostwald-Reifung, so dass die bereits weiter oben gegebenen Bedingungen erfüllt sind.

Abschließend erfolgt eine weitere Aufnahme der $M_{MAX}(\sigma)$-Kurve. Das Maximum dieser aufgenommenen Kurve wird einer dritten Lastspannung $\sigma_3$ zugeordnet.

Aus den erhaltenen Lastspannungen $\sigma_1$, $\sigma_2$ und $\sigma_3$ lassen sich nunmehr die Mikroeigenspannungen II. sowie III. Art ableiten.

Dabei wird die Mikroeigenspannung II. Art als Differenz $\sigma_{therm\ II} = |\sigma_1 - \sigma_3|$ ermittelt.

Zudem lässt sich die Mikroeigenspannung III. Art als Differenz $\sigma_{Koh.\ III} = |\Delta\sigma - \sigma_{therm\ II}|$ bestimmen, wobei für die Differenz $\Delta\sigma$ gilt: $\Delta\sigma = \sigma_{therm\ II} + \sigma_{Koh\cdot\ III} = |\sigma_1 - \sigma_2|$.

Alternativ ist die Bestimmung der Mikroeigenspannung III. Art als Differenz der zuvor ermittelten Werte $\sigma_2$ und $\sigma_3$ möglich:

$$\sigma_{Koh.\ III} = |\sigma_3 - \sigma_2|.$$

Das im Voranstehenden beschriebene Verfahren bedarf daher lediglich zweier Auslagerungsvorgänge sowie einer dreimaligen Aufnahme von $M_{MAX}(\sigma)$-Kurven, um sowohl die Mikroeigenspannung II. als auch III. Art ermitteln zu können.

Besonderer Vorteil der beiden zuvor beschriebenen erfindungsgemäßen Verfahren ist dabei, dass die Verfahren einfach durchführbar, reproduzierbar als auch zerstörungsfrei ablaufen. Zudem werden keine Referenzverfahren benötigt.

Der wichtigste Vorteil des erfindungsgemäßen Prüfungsverfahrens besteht darin, dass er eine zerstörungsfreie und quantitative Ermittlung von Mikroeigenspannungen II. und III. Art sowie eine Separation dieser beiden Mikroeigenspannungen erlaubt.

Der entwickelte Prüfverfahrensansatz ermöglicht z.B. eine Online-Überwachung der Mikroeigenspannungsänderungen von Komponenten (z.B. Reaktordruckbehältern, Rohrleitungen aus Stählen basierend auf Fe-Cu-Systemen).

Beim erfindungsgemäßen Verfahren zur Bestimmung der Mikrospannung III. und/oder II. Art ist dabei bevorzugt, wenn die erste Auslagerung des Prüfkörpers durch Lösungsglühen des Prüfkörpers, Abschrecken des Prüfkörpers sowie Wärmebehandlung des Prüfkörpers erfolgt.

Zudem ist bevorzugt, wenn

a) das Lösungsglühen des Prüfkörpers bei Temperaturen zwischen 700 und 911 °C, bevorzugt zwischen 750 und 905 °C, insbesondere zwischen 800 und 860 °C und/oder über einen Zeitraum von 30 min bis 24 Stunden, bevorzugt von 30 min bis 5 Stunden, insbesondere von 1 bis 3 Stunden erfolgt,

b) das Abschrecken durch Eintauchen des Prüfkörpers in ein Fluid, insbesondere Wasser erfolgt, und/oder

c) die Wärmebehandlung durch ein oder mehrstufiges Lagern des Prüfkörpers bei Temperaturen zwischen 250 und 750 °C, bevorzugt zwischen 300 und 600 °C, insbesondere zwischen 350 und 550 °C und/oder über einen Zeitraum von bis zu 14 Stunden, bevorzugt von 5 Stunden bis 14 Stunden erfolgt.

Beim erfindungsgemäßen Verfahren ist weiterhin vorteilhaft, wenn die weitere Auslagerung des Prüfkörpers bis zur Ostwald-Reifung durch thermische Überalterung des Prüfkörpers im Mehrphasenbereich erfolgt.

Vorzugsweise erfolgt die thermische Überalterung durch ein oder mehrstufiges Lagern des Prüfkörpers bei Temperaturen zwischen 250 und 750°C, bevorzugt zwischen 300 und 600 °C, insbesondere zwischen 350 und 550 °C und/oder über einen Zeitraum von 14 Stunden bis 108 Stunden.

Da die thermische Aus- und Überalterung diffusionsgesteuert ablaufen, hängen die Zeiten und Temperaturen bei diesen Vorgängen vom Cu-Gehalt der Legierung ab.

Erfindungsgemäß wird ebenso ein Verfahren zur zerstörungsfreien quantitativen Bestimmung der Mikroeigenspannung II. Art angegeben, das ebenso auf den zuvor erkannten Prinzipien beruht. Gemäß diesem Verfahren, das an einem Fe- und Cu-haltigen Legierungssystem mit einem Cu-Gehalt $\leq 2$ Gew.-% durchgeführt werden kann, werden die folgenden Schritte durchgeführt:

a) Bestimmung des Lastspannungswertes $\sigma_1$, bei dem die Lastspannungsabhängigkeit der maximalen Barkhausenrauschamplitude $M_{MAX}(\sigma)$ für einen Prüfkörper aus dem Legierungssystem in einem Ausgangszustand ein Maximum aufweist,

b) Auslagerung des Prüfkörpers bis zur Ostwald-Reifung,

c) Bestimmung des Lastspannungswertes $\sigma_3$, bei dem die Lastspannungsabhängigkeit der maximalen Barkhausenrauschamplitude $M_{MAX}(\sigma)$ für einen Prüfkörper nach der Auslagerung bis zur Ostwald-Reifung und Abkühlung einen maximalen Wert aufweist, sowie

d) Ermittlung der Mikroeigenspannung II. Art als Differenz $\sigma_{therm. II} = |\sigma_1 - \sigma_3|$.

Die Bestimmung der Lastspannungswerte $\sigma_1$ und $\sigma_3$ im Ausgangszustand bzw. im ausgelagerten Zustand (bis zur Ostwald-Reifung) erfolgt dabei gemäß den vorangehend beschriebenen Prinzipien.

Bei der Auslagerung des Prüfkörpers bis zur Ostwald-Reifung, ausgehend vom Ausgangszustand, wird eine thermische Auslagerung durchgeführt, der den Prüfkörper vom Ausgangszustand in den Zustand der Ostwald-Reifung überführt.

In einer bevorzugten Ausführungsform erfolgt die Auslagerung des Prüfkörpers bis zur Ostwald-Reifung durch Wärmebehandlung, bei dem ein Lösungsglühen des Prüfkörpers, Abschrecken des Prüfkörpers sowie thermischer Überalterung des Prüfkörpers bis zur Ostwald-Reifung durchgeführt wird.

Zum Beispiel kann das Lösungsglühen des Prüfkörpers bei Temperaturen zwischen 700 und 911 °C, bevorzugt zwischen 750 und 905 °C, insbesondere zwischen 800 und 860 °C und/oder über einen Zeitraum von 30 min bis 24 Stunden, bevorzugt von 30 min bis 5 Stunden, insbesondere von 1 bis 3 Stunden ausgeführt werden. Die Wahl der Temperatur und/oder des Zeitraums kann dabei nach Kupfergehalt der Legierungssysteme gewählt werden.

Das Abschrecken des Prüfkörpers erfolgt bevorzugt durch Eintauchen des Prüfkörpers in ein Fluid, insbesondere Wasser.

Die thermische Überalterung erfolgt bevorzugt durch ein oder mehrstufiges Lagern des Prüfkörpers bei Temperaturen zwischen 250 und 750 °C, bevorzugt zwischen 300 und 700 °C, insbesondere zwischen 350 und 550 °C und/oder über einen Zeitraum von bis zu 108 Stunden, bevorzugt von 5 Stunden bis 108 Stunden erfolgen.

Weiterhin stellt die vorliegende Erfindung ebenso ein Verfahren zur zerstörungsfreien quantitativen Bestimmung der Mikroeigenspannung III. Art (Kohärenz-Zugeigenspannung III. Art) eines Fe- und Cu-haltigen Legierungssystems, mit einem Cu-Gehalt $\leq 2$ Gew.-%, bereit, umfassend folgende Schritte:

a) erste Auslagerung des Prüfkörpers bis zur ausschließlichen Bildung von kohärenten Cu-Ausscheidungen,

b) Bestimmung des Lastspannungswertes $\sigma_2$, bei dem die Lastspannungsabhängigkeit der maximalen Wertes der Barkhausenrauschamplitude $M_{MAX}(\sigma)$ für einen Prüfkörper nach der ersten Auslagerung und Abkühlung ein Maximum aufweist.

c) weitere Auslagerung des Prüfkörpers bis zur Ostwald-Reifung,

d) Bestimmung des Lastspannungswertes $\sigma_3$, bei dem die Lastspannungsabhängigkeit der Barkhausenrauschamplitude $M_{MAX}(\sigma)$ für einen Prüfkörper nach der weiteren Auslagerung bis zur Ostwald-Reifung und Abkühlung ein Maximum aufweist, sowie

e) Ermittlung der Mikroeigenspannung III. Art als Differenz $\sigma_{Koh. III} = |\sigma_3 - \sigma_2|$.

Dieses Verfahren wird im Wesentlichen wie das zuvor beschriebene Verfahren (das Verfahren gemäß Anspruch 1) durchgeführt, allerdings ist hierbei die Bestimmung des maximalen Wertes der Lastspannungsabhängigkeit des maximalen Wertes der Barkhausenrauschamplitude im Ausgangszustand des Prüfkörpers nicht notwendig.

Für alle zuvor beschriebenen Aspekte der vorliegenden Erfindung, d.h. sowohl für das Verfahren zur gleichzeitigen Bestimmung der Mikroeigenspannungen II. und III. Art, als auch für die Verfahren, mit denen lediglich die Mikroeigenspannungen II. oder III. Art bestimmt werden können, gelten die nachfolgend bevorzugten Ausführungsformen:

Bevorzugte, einsetzbare Legierungssysteme für den Prüfkörper sind dabei ausgewählt aus der Gruppe bestehend aus Fe-Cu-, Fe-Cu-Ni- oder Fe-Cu-Ni-Mn-Legierungen. Die Legierungen sind dabei ausscheidungsfähig, d.h. es kann bei thermischer Behandlung der Legierungen Cu ausgeschieden werden.

Für alle zuvor genannten Legierungssysteme ist es bevorzugt, wenn der Cu-Mindestgehalt 0,1 Gew.-%, bevorzugt 0,3 Gew.-%, beträgt.

Für den Fall, dass eine Fe-Cu-Legierung verwendet wird, ist es bevorzugt, wenn diese Cu-Gehalt von 0,1 bis 2 Gew.-%, bevorzugt von 0,3 bis 2 Gew.-%, insbesondere von 0,6 bis 2 Gew.-% aufweist.

Bevorzugte Fe-Cu-Ni-Legierungssysteme weisen dabei einen Cu-Gehalt von 0,1 bis 2 Gew.-%, bevorzugt von 0,3 bis 2 Gew.-%, insbesondere von 0,6 bis 2 Gew.-%; sowie einen Ni-Gehalt von 0,1 bis 10 Gew.-%, bevorzugt von 0,5 bis 5 Gew.-%, insbesondere von 0,8 bis 2 Gew.-% auf.

Die Erfindung ist ebenso bei Fe-Cu-Ni-Mn-Legierungssystemen einsetzbar, die vorteilhafterweise einen Cu-Gehalt von 0,1 bis 2 Gew.-%, bevorzugt von 0,3 bis 2 Gew.-%, insbesondere von 0,6 bis 2 Gew.-%; einen Ni-Gehalt von von 0,1 bis 10 Gew.-%, bevorzugt von 0,5 bis 5 Gew.-%, insbesondere von 0,8 bis 2 Gew.-%; sowie einen Mn-Gehalt von 0,1 bis 8 Gew.-%, bevorzugt von 0,3 bis 5 Gew.-%, insbesondere von 0,5 bis 1,3 Gew.-% aufweisen.

Bei den zuvor genannten beispielhaften Legierungssystemen können die Einzelbestandteile jeweils unabhängig von den anderen Bestandteilen gewählt werden. Die Eisenmatrix liegt dabei additiv zu den genannten Komponenten vor, so dass sich 100 Gew.-% ergeben.

Bei den erfindungsgemäßen Verfahren ist es bevorzugt, wenn die an den Prüfkörper angelegte maximale Lastspannung höchstens 50 % der Streckgrenze des Materials des Prüfkörpers beträgt.

Zudem ist es vorteilhaft, wenn die an den Prüfkörper angelegte Lastspannung $\sigma$ von 0,01 bis 100 MPa, bevorzugt von 0,1 bis 50 MPa variiert wird.

Insbesondere werden an den Prüfkörper Zuglastspannungen angelegt.

Die vorliegende Erfindung wird anhand der nachfolgenden Ausführungen sowie Figuren näher erläutert, ohne die Erfindung auf die dargestellten Parameter zu beschränken.

Das erfindungsgemäße Verfahren wird nachfolgend anhand des Beispiels eines Verfahrens zur Bestimmung der Mikroeigenspannungen III. Art bei gleichzeitiger Bestimmung der Mikroeigenspannung II. Art näher ausgeführt.

Die Ausführungsformen sind jedoch in analoger Weise auf die ebenso erfindungsgemäße Ausführungsform übertragbar, bei der lediglich eine Bestimmung der Mikroeigenspannung II. Art oder III. Art erfolgt.

Das erfindungsgemäße Prüfverfahren erfordert die Aufnahmevon $M_{MAX}(\sigma)$-Kurven für drei Wärmebehandlungszustände: den Ausgangszustand (abgeschreckt noch nicht ausgelagert), den bis ins Härtemaximum ausgelagerten Zustand sowie den bis ins Gebiet der Ostwald-Reifung ausgelagerten Zustand.

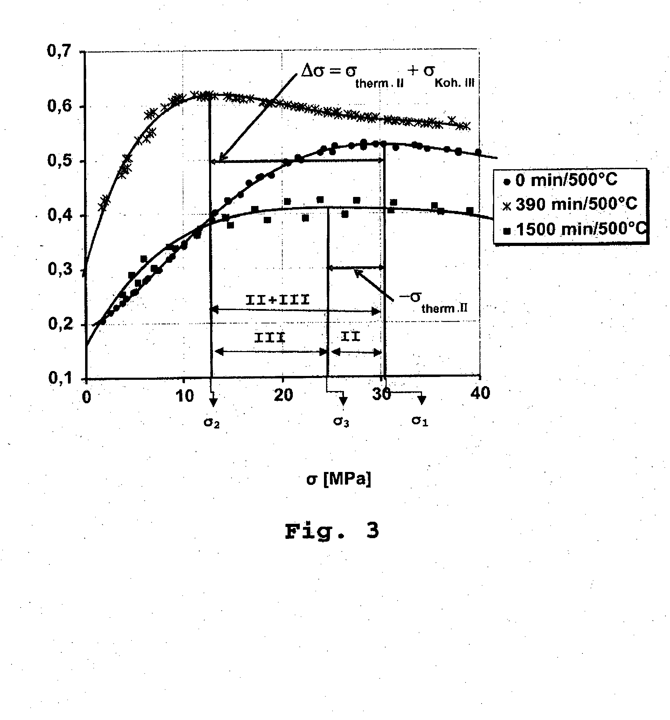

Figur 3 zeigt die maximale Lastspannungsabhängigkeit der maximale Barkhausenrauschamplitude ($M_{MAX}(\sigma)$-Kurven) als Funktion von Zuglastspannungen für diese drei Wärmebehandlungszustande beispielhaft für eine Fe-Cu-Legierung mit 1,0 Gew.-% Cu. Diese $M_{MAX}(\sigma)$-Kurven unterscheiden sich durch die Lage ihres Maximums auf der Zuglastspannungsachse. Bezüglich der Maximums-Verschiebungen der $M_{MAX}(\sigma)$-Kurven wurden im Rahmen früherer Arbeiten (vgl. I. Altpeter et al. (NDT&E International 42 (2009), 283-290)) folgende Beobachtungen gemacht: Druckeigenspannungen führen im Vergleich zum Ausgangszustand zu einer Verschiebung des Maximums hin zu höheren Zuglastspannungswerten, während Zugeigenspannungen eine Verschiebung des Maximums hin zu geringeren Zuglastspannungswerten bewirken. Somit wird über die Maximums-Verschiebung direkt die Änderung des Eigenspannungszustandes charakterisiert.

Der oben genannte Ausgangszustand ist im Falle der Fe-Cu-Legierungen der abgeschreckte, nicht ausgelagerte Zustand. Dieser Zustand wird durch die beim Abschrecken thermisch induzierten Druckeigenspannungen I. Art charakterisiert (Figur 3 - 0 min).

Während der Auslagerung der Fe-Cu-Proben laufen zwei gegenläufige Prozesse ab. Zu Beginn der Auslagerung werden die durch die Probenherstellung entstandenen Abschreck-Druckeigenspannungen (thermisch induzierten Eigenspannungen I. Art) abgebaut. Mit zunehmender Auslagerungszeit nehmen die sog. Kohärenz-Zugeigenspannungen III. Art herrührend von den Cu-Ausscheidungen in der $\alpha$-Fe-Matrix zu. Durch die Abkühlung nach der Auslagerung entstehen zusätzliche thermisch induzierte Druckeigenspannungen I. und II. Art. Die Peakverschiebung zwischen dem Ausgangszustand und dem bei 500°C 390 Minuten ausgelagerten Zustand entspricht einer Eigenspannungsänderung von $\Delta\sigma = \sigma_{therm\,II} + \sigma_{Koh.\,III.}$ Um die Kohärenz-Zugeigenspannungen III. Art bestimmen zu können, kann bevorzugt folgende Vorgehensweise durchgeführt werden:

Schritt 1) Aufnahme der $M_{MAX}(\sigma)$-Kurven des Ausgangszustandes nach dem Abschrecken und Bestimmung der Lage des Maximums bei $\sigma_1$.

Schritt 2) Aufnahme der $M_{MAX}(\sigma)$-Kurve nach einer Auslagerungszeit von 390 Minuten bei 500 °C, anschließender Abkühlung und Bestimmung der Lage des Maximums bei $\sigma_2$. Diese Auslagerungszeit wurde ausgehend von den Härte- und SANS-Messungen so gewählt, dass ausschließlich kohärente Cu-Ausscheidungen in der Eisenmatrix vorliegen (Figur 3 - 390 min) Dieser Zustand ist durch thermisch induzierte Druckeigenspannungen I. Art, thermisch induzierte Druckeigenspannungen II. Art und Kohärenz-Zugeigenspannungen III. Art charakterisiert. Die Verschiebung $\Delta\sigma$ des Maximums der $M_{MAX}(\sigma)$-Kurve zwischen dem abgeschreckten noch nicht ausgelagerten Zustand und dem ausgelagerten abgeschreckten Zustand beträgt $\Delta\sigma = \sigma_{therm\,II} + \sigma_{Koh.\,III} = 17$ MPa. Dieser relativ kleine Betrag ist auf eine Summe der thermisch induzierten Mikrodruckeigenspannungen II. Art und der Kohärenz-Zugeigenspannungen III. Art zurückzuführen. Um die Kohärenz-Zugeigenspannungen III. Art bestimmen zu können, müssen die thermisch induzierten Mikrodruckeigenspannungen II. Art bekannt sein.

Schritt 3) Aufnahme der $M_{MAX}(\sigma)$-Kurve nach einer Auslagerung im Gebiet der Ostwald-Reifung (nach 1500 min bei 500°C) und anschließender Abkühlung (Figur 3 - 1500 min) und Bestimmung der Lage des Maximums bei $\sigma_3$. Dieser Zustand ist durch thermisch induzierte Druckeigenspannungen I. Art und thermisch induzierte Druckeigenspannungen II. Art charakterisiert, da durch die Ostwald-Reifung die Cu-Ausscheidungen inkohärent werden und somit keine Kohärenz-Zugeigenspannungen mehr vorliegen. Die Verschiebung des Maximums der $M_{MAX}(\sigma)$-Kurve zwischen dem abgeschreckten noch nicht ausgelagerten Zustand und dem Ostwaldgereiften abgeschreckten Zustand entspricht den thermisch induzierten Druckeigenspannungen II. Art und beträgt 5 MPa.

Schritt 4) Bestimmung der Kohärenz-Zugeigenspannungen III. Art durch das Subtrahieren der thermisch induzierten Eigenspannungen II. Art von der Summe $\Delta\sigma = \sigma_{therm\,II} + \sigma_{Koh.\,III}$. Dies bedeutet für die in Figur 3 gezeigte Legierung einen Kohärenz-Zugeigenspannungsbetrag von 22 MPa.

Die bevorzugten Legierungen, an denen mittels dieses Verfahrens die Mikroeigenspannungen II. und III. Art zerstörungsfrei bestimmt werden können, sind ausscheidungsfähige Fe-Cu-Legierungen. Beispielhafte ausscheidungsfähigen Fe-Cu-Legierungen enthaltenen maximal 2,0 Gew-% Cu und Rest Fe. Im Rahmen der erfindungsgemäßen Erfindung wurden Fe-Cu-Legierungen mit 0.65, 0.75, 0.9, 1.0, 1.2, 1.4, 1.5, 1.7 und 1.9 Gew.-% Cu hergestellt und untersucht.

Weitere bevorzugte Legierungen, an denen mittels dieses Verfahrens die Mikroeigenspannungen II. und III. Art zerstörungsfrei bestimmt werden können, sind ausscheidungsfähige Fe-Cu-Ni-Mn-Legierungen. Die untersuchten Fe-Cu-Ni-Mn-Legierungen enthalten 0.65 Gew.-% Cu, 1.0 Gew.-% Ni mit jeweils 0.75, 0.95, 1.15 und 1.3 Gew.-%Mn sowie 1.3 Gew.-% Ni mit jeweils 0.75, 0.95, 1.15 und 1.3 Gew.-% Mn.

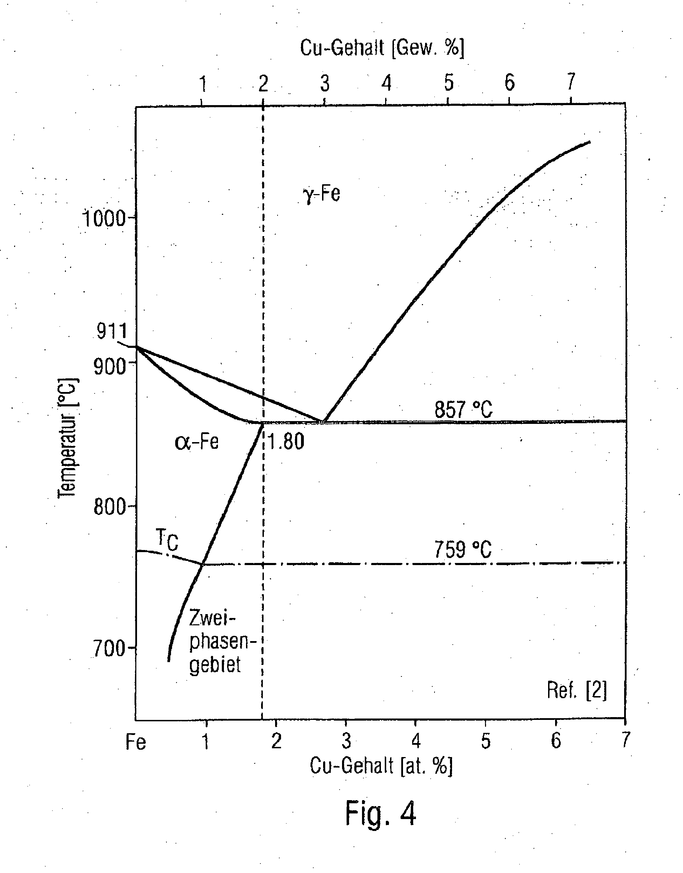

Die Fe-Cu-Legierungen werden im Einphasenbereich (Figur 4) bei 850°C 2 Stunden lang lösungsgeglüht, in Wasser abgeschreckt und anschließend thermisch im Zweiphasenbereich ausgelagert (unterhalb der Löslichkeitslinie siehe Figur 4) bei 500°C bis zu 1500 Minuten lang. Um Proben mit einem abgestuften Volumenanteil an kohärenten Cu-Ausscheidungen herzustellen, wurden sie unterschiedlich lang, abhängig vom Cu-Gehalt ausgelagert. In jedem Härteverlauf spiegelt sich die Umwandlung von kohärenten über teilkohärente in inkohärente Ausscheidungen während der Auslagerung wider. Ein typischer Härteverlauf zeichnet sich durch einen ansteigenden Ast aus, in welchem eine Legierung mit hauptsächlich kohärenten Ausscheidungen vorliegt, ein Härtemaximum, in dem teilkohärente Ausscheidungen überwiegen und einen abnehmenden Ast, in dem inkohärente Ausscheidungen vorliegen und in dem Ostwald-Reifungsprozesse stattfinden. Um die zur Einstellung von kohärenten Cu-Ausscheidungen ausreichende Auslagerungszeit zu bestimmen, wurden zuerst Testproben hergestellt. An diesen Testproben wurden bei 500 °C in Zeitschritten unterschiedlicher Länge, abhängig vom Cu-Gehalt, Härtemessungen (nach Vickers, DIN EN ISO 6507-4:2005) durchgeführt. Dieses Experiment wurde solange durchgeführt, bis das Härtemaximum erreicht wurde. Um Legierungen mit kohärenten Ausscheidungen herzustellen, sollten die Auslagerungsdauern abhängig vom jeweiligen Cu-Gehalt unterhalb der Wärmebehandlungsdauer gewählt werden, die dem jeweiligen Härtemaximum entspricht. Auf diese Weise wurde die notwendige Auslagerungsdauer, um ausschließlich kleinen (1-1.5 nm Radius), kohärente Ausscheidungszustände einzustellen, bestimmt (Tabelle 2). In einem ersten Auslagerungsschritt wurden Ausscheidungen mit mittleren Radien von ca. 1 nm und in einem zweiten Auslagerungsschritt wurden Ausscheidungen mit mittleren Radien von ca. 1.5 nm eingestellt. Die Neutronenkleinwinkelstreuung (engl. SANS)-Untersuchungen haben gezeigt, dass die hergestellten Proben Cu-Ausscheidungen mit einem Radius kleiner 5 nm aufweisen. Hieraus kann geschlossen werden, dass in den Fe-Cu-Legierungen tatsächlich kohärente Cu-Ausscheidungen vorliegen. Somit wurde bewiesen, dass die gewählten Auslagerungszeiten ausreichend sind, um kleine (1-1.5 nm Radius - siehe Tabelle 3) kohärente Cu-Ausscheidungen einzustellen.

Tabelle 2: Auslagerungsdauer zur Einstellung kohärenter Cu-Ausscheidungen

| Gew.% Cu | 0,5 | 0,65 | 0,75 | 0,9 | 1,0 | 1,2 | 1,4 | 1,5 | 1,7 | 1,9 |
|---|---|---|---|---|---|---|---|---|---|---|
| Auslagerungsdauer [h] | 35 | 14 | 10 | 7,83 | 6,5 | 4,5 | 3,5 | 3 | 1,5 | 1,17 |

Tabelle 3: Messergebnisse aus den SANS-Untersuchungen abgeleitet

| Cu-Gehalt [Gew.%] | Cu-Auscheidungsradius [nm] | Anteil an ausgeschiedenen Cu-Teilchen [Vol.%] | Dichte [$cm^{-3}$] |
|---|---|---|---|
| 0.65 | 0.81 | 0.112 | $2.27 \times 10^{17}$ |
| 1.0 | 0.81 | 0.347 | $9.35 \times 10^{17}$ |
| 1.4 | 1.17 | 0.729 | $9:2.6 \times 10^{17}$ |
| 1.7 | 1.06 | 0.676 | $1.34 \times 10^{18}$ |

Anschließend wurden die Proben thermisch überaltert (thermisch ausgelagert bis in den Bereich der Ostwald-Reifung. Im Stadium der Ostwald-Reifung bleibt der ausgeschiedene Volumenanteil konstant, während der mittlere Teilchendurchmesser und der Teilchenabstand zunehmen. Dies wird dadurch ermöglicht, dass kleinere Teilchen von größeren aufgezehrt werden, so dass sich die größeren Teilchen vergröbern. Da die Teilchen selbst nicht wandern können, werden auch diese Vorgänge durch atomare Diffusion gesteuert.

Die Fe-Cu-Ni-Mn-Legierungen werden bspw. im Einphasenbereich (Figur 4) bei 850°C 2 Stunden lang lösungsgeglüht, abgeschreckt im Wasser und anschließend thermisch ausgelagert im Zweiphasenbereich (unterhalb der Löslichkeitslinie) bei 500°C 12 Stunden lang und danach bei 360°C bis zu 181 Tage.

Diese Auslagerungszeiten wurden mittels Simulationsrechnungen so berechnet, dass die Legierungen nanoskalige Cu-Ausscheidungen ummantelt von einer Ni-Mn-Schale (wie in den Cu-haltigen Strahlen) enthalten.

Im Falle der Fe-Cu-Ni-Mn-Legierungen wurde die Änderung des Mikroeigenspannungszustandes ebenfalls durch Messung der $M_{MAX}(\sigma)$-Kurven unterschiedlicher Auslagerungszustände charakterisiert.

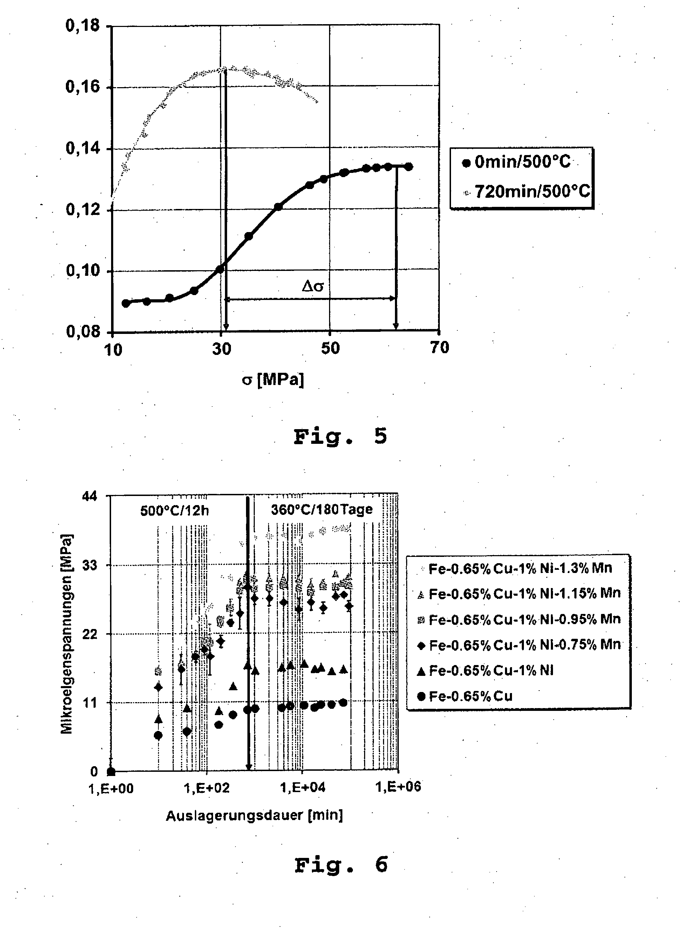

Figur 5 zeigt die $M_{MAX}(\sigma)$-Kurve für die Fe-0.65 Gew.-% Cu-1.0-Gew.-% Ni-0.75 Gew.-% Mn-Legierung im Ausgangszustand und im endgültig ausgelagertem Zustand (Ostwald-Reifung). Im Falle dieser Fe-Cu-Ni-Mn-Legierungen wurde nach erfolgter Auslagerung eine Verschiebung des Maximums der $M_{MAX}(\sigma)$-Kurven zu kleineren Zuglastspannungswerten hin gemessen. Dies deutet auf eine durch die Auslagerung bedingt induzierte Zunahme der Mikroeigenspannungen II. und III. Art bis zu 40 MPa hin, da diese Proben so ausgelagert wurden, dass ausschließlich kohärente Ausscheidungen entstehen.

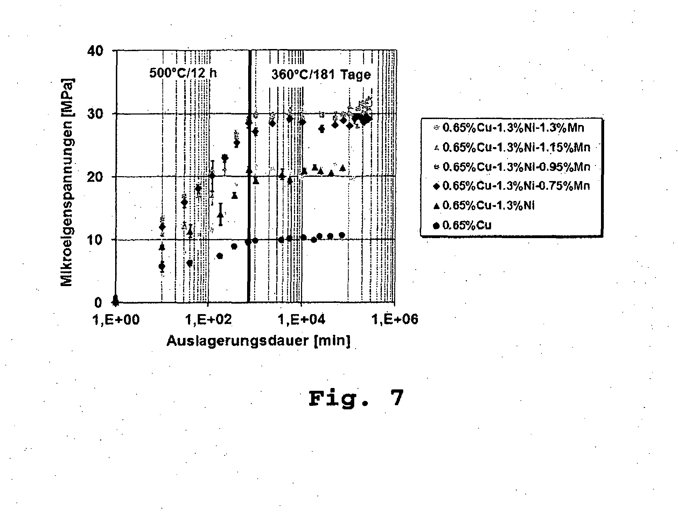

Figur 6 zeigt den Verlauf der aus den $M_{MA}(\sigma)$-Kurven abgeleiteten Mikroeigenspannungswerte ($\Delta\sigma$) als Funktion der Auslagerungsdauer. Es wurde festgestellt, dass im Fall der Proben mit 1.0 Gew.-% Ni eine Zunahme des Mn-Gehaltes zu einer Zunahme der Mikroeigenspannungen führt, obwohl der Cu-Gehalt konstant bleibt. In Figur 7 kann beobachtet werden, dass allein das Zulegieren von Ni eine Zunahme von bis zu 18 MPa bewirkt. Das zusätzliche Zulegieren von Mn führt zu einer weiteren Zunahme der Mikroeigenspannungen bis auf 40 MPa.

Neben den experimentellen Untersuchungen wurden atomistische Monte-Carlo (MC)-Simulationen zur Bildung und zum Wachstum von Ausscheidungen sowie Molekulardynamik (MD)-Simulationen zur Bestimmung der Eigenspannungen in oben genannten Legierungen durchgeführt. Für die MC-Simulationen wurde ein verbesserter, praxistauglicher energetischer Ansatz verwendet, der auf quantenmechanischen ab-initio-Berechnungen beruht. Die MC-Simulationen liefern die mittleren Radien, die Anzahldichte und die chemische Zusammensetzung der Ausscheidungen bei unterschiedlichen Ni- und Mn-Gehalten sowie bei unterschiedlichen Temperaturbehandlungen. In zusätzlichen MC-Simulationen wurde gezeigt, dass sowohl Ni als auch Mn die Anzahldichte der Ausscheidungen erhöhen.

Für die MD-Simulationen wurde ein neues, 2009 veröffentlichtes Fe-Cu-Ni-EAM (Embedded Atom Method)-Potential implementiert und verwendet, sowie ein Auswerteprogramm zur Bestimmung der lokalen als auch der globalen Spannungen entwickelt. Die MD-Simulationen zeigen, dass an Fe/Cu-Grenzflächen sehr hohe lokale Spannungen bis zu 3000 MPa auftreten, die im Inneren der Cu-Ausscheidung deutlich sinken. Für die aus den MC-Simulationen erhaltenen Fe-Cu- und Fe-Cu-Ni-Ausscheidungszustande wurden MD-Simulationen (Relaxationen, anschlieBend Number pressure Temperature-Ensemble) für verschiedene Temperaturen durchgeführt und die globalen Eigenspannungen für jede Atomsorte berechnet. Hiermit wird eine Unterscheidung zwischen Kohärenz-und thermisch induzierten Eigenspannungen ermöglicht. Aus den MD-Simulationen ergeben sich für die Proben mit 0.65 Gew.-% Cu, 1.0 Gew.-% Ni und unterschiedlichen Mn-Gehalten bei steigendem Mn-Anteil bis zu 40 MPa durch die Ausscheidungen verursachte Zugeigenspannungen in der Fe-Matrix. Die Simulationen stehen in guter Übereinstimmung mit den am IZFP durchgeführten Experimenten.

Auch für die Proben mit 0.65 Gew.-% Cu, 1.3 Gew-% Ni und unterschiedlichen Mn-Gehalten ergaben sich gute Übereinstimmungen zwischen experimentellen und Simulationsergebnissen bzgl. Der Mikroeigenspannungen (s. hierzu Tabelle 4 und Figur 7)

Tabelle 4: Vergleich zwischen experimentellen und Simulations-Ergebnissen, $\Delta\sigma$ ist jeweils die Differenz zwischen Ausscheidungszustand und Mischkristall

| | 12h, 500°C | | 12h, 500°C, dann 180 Tage, 360°C | |
|---|---|---|---|---|
| | $\Delta\sigma$ [MPa'] | | $\Delta\sigma$ [MPa] | |
| | Experiment | Simulation | Experiment | Simulation |
| Fe-0.65 Gew.%Cu | 10 | 18 | 11 | 18 |
| Fe-0.65 Gew.%Cu-1.30 Gew.%Ni | 21 | 19 | 22 | 22 |
| Fe-0.65 Gew.%Cu-1.30 Gew.%Ni-0.75 Gew.%Mn | 28 | 25 | 29 | 32 |
| Fe-O.65 Gew.%Cu-1.30 Gew.%Ni-0.95 Gew. -%Mn | 29 | 26 | 31 | 36 |
| Fe-0.65 Gew.%Cu-1.30 Gew. %Ni-1.15 Gew.%Mn | 29 | 27 | 28 | 38 |
| Fe-0.65 Gew.%Cu-1.30 Gew.%Ni-1.30 Gew.%Mn | 21 | 29 | 19 | 18 |

**Patentansprüche**

1.  Verfahren zur zerstörungsfreien quantitativen Bestimmung der Mikroeigenspannung II. Art (thermisch induzierte Eigenspannung II. Art) eines Fe- und Cu-haltigen Legierungssystems mit einem Cu-Gehalt $\leq 2$ Gew.-% umfassend folgende Schritte:

    a) Bestimmung des Lastspannungswertes $\sigma_1$, bei dem die Lastspannungsabhängigkeit der maximalen Barkhausenrauschamplitude $M_{MAX}(\sigma)$ für einen Prüfkörper aus dem Legierungssystem in einem Ausgangszustand, ein Maximum aufweist,
    b) Auslagerung des Prüfkörpers bis zur Ostwald-Reifung,
    c) Bestimmung des Lastspannungswertes $\sigma_3$, bei dem die Lastspannungsabhängigkeit der maximalen Barkhausenrauschamplitude $M_{MAX}(\sigma)$ für einen Prüfkörper nach der Auslagerung bis zur Ostwald-Reifung und Abkühlung einen maximalen Wert aufweist, sowie
    d) Ermittlung der Mikroeigenspannung II. Art als Differenz $\sigma_{therm. II} = |\sigma_1 - \sigma_3|$.

2.  Verfahren nach vorhergehendem Anspruch, **dadurch gekennzeichnet, dass** die Auslagerung des Prüfkörpers bis zur Ostwald-Reifung durch Lösungsglühen des Prüfkörpers, Abschrecken des Prüfkörpers sowie thermischer Überalterung des Prüfkörpers erfolgt.

3.  Verfahren nach vorhergehendem Anspruch, **dadurch gekennzeichnet, dass** das Lösungsglühen im Löslichkeitsbereich und/oder die thermische Überalterung im Mehrphasenbereich erfolgt.

4.  Verfahren nach einem der beiden vorhergehenden Ansprüche, **dadurch gekennzeichnet, dass**

    a) das Lösungsglühen des Prüfkörpers bei Temperaturen zwischen 700 und 911 °C, bevorzugt zwischen 750 und 905 °C, insbesondere zwischen 800 und 860 °C und/oder über einen Zeitraum von 10 min bis 24 Stunden, bevorzugt von 30 min bis 5 Stunden, insbesondere von 1 bis 3 Stunden erfolgt,
b) das Abschrecken durch Eintauchen des Prüfkörpers in ein Fluid, insbesondere Wasser erfolgt, und/oder
c) die thermische Überalterung durch ein oder mehrstufiges Lagern des Prüfkörpers bei Temperaturen zwischen 250 und 750 °C, bevorzugt zwischen 300 und 600 °C, insbesondere zwischen 350 und 550 °C und/oder über einen Zeitraum von bis zu 108 Stunden, bevorzugt von 5 Stunden bis 108 STunden erfolgt.

5. Verfahren zur zerstörungsfreien quantitativen Bestimmung der Mikroeigenspannung III. Art (Kohärenz-Zugeigenspannung III. Art) eines Fe- und Cu-haltigen Legierungssystems, mit einem Cu-Gehalt $\leq$ 2 Gew.-%, umfassend folgende Schritte:

a) erste Auslagerung des Prüfkörpers bis zur ausschließlichen Bildung von kohärenten Cu-Ausscheidungen,
b) Bestimmung des Lastspannungswertes $\sigma_2$, bei dem die Lastspannungsabhängigkeit der maximalen Wertes der Barkhausenrauschamplitude $M_{MAX}(\sigma)$ für einen Prüfkörper nach der ersten Auslagerung und Abkühlung ein Maximum aufweist.
c) weitere Auslagerung des Prüfkörpers bis zur Ostwald-Reifung,
d) Bestimmung des Lastspannungswertes $\sigma_3$, bei dem die Lastspannungsabhängigkeit der Barkhausenrauschamplitude $M_{MAX}(\sigma)$ für einen Prüfkörper nach der weiteren Auslagerung bis zur Ostwald-Reifung und Abkühlung ein Maximum aufweist, sowie
e) Ermittlung der Mikroeigenspannung III. Art als Differenz $\sigma_{Koh.\ III} = |\sigma_3 - \sigma_2|$.

6. Verfahren nach einem der vorhergehenden Ansprüche, **dadurch gekennzeichnet, dass** es zur zerstörungsfreien quantitativen Bestimmung der Mikroeigenspannung III. Art (Kohärenz-Zugeigenspannung III. Art, $\sigma_{Koh.\ III}$) und II. Art (thermisch induzierte Eigenspannung II. Art, $\sigma_{therm.\ II}$) und/oder der Summe der Mikroeigenspannungen II. und III. Art ($\Delta\sigma = \sigma_{therm.\ II} + \sigma_{Koh.\ III}$) eines Fe- und Cu-haltigen Legierungssystems mit einem Cu-Gehalt von $\leq$ 2 Gew.-% dient und folgende Schritte umfasst:

a) Bestimmung des Lastspannungswertes $\sigma_1$, bei dem die Lastspannungsabhängigkeit der maximalen Barkhausenrauschamplitude $M_{MAX}(\sigma)$ für einen Prüfkörper aus dem Legierungssystem in einem Ausgangszustand, ein Maximum aufweist,
b) erste Auslagerung des Prüfkörpers bis zur ausschließlichen Bildung von kohärenten Cu-Ausscheidungen,
c) Bestimmung des Lastspannungswertes $\sigma_2$, bei dem die Lastspannungsabhängigkeit der maximalen Barkhausenrauschamplitude $M_{MAX}(\sigma)$ für einen Prüfkörper nach der ersten Auslagerung und Abkühlung ein Maximum aufweist
d) weitere Auslagerung des Prüfkörpers bis zur Ostwald-Reifung,
e) Bestimmung des Lastspannungswertes $\sigma_3$, bei dem die Lastspannungsabhängigkeit der maximalen Barkhausenrauschamplitude $M_{MAX}(\sigma)$ für einen Prüfkörper nach der weiteren Auslagerung bis zur Ostwald-Reifung und Abkühlung ein Maximum aufweist,
f) Ermittlung der Mikroeigenspannung II. Art ($\sigma_{therm.\ II}$) als Differenz $\sigma_{therm.\ II} = |\sigma_1 - \sigma_3|$
g) Ermittlung der Mikroeigenspannung III. Art durch

i. Bilden der Summe der Mikroeigenspannung II. Art und der Mikroeigenspannung III. Art als Differenz

$$\Delta\sigma = \sigma_{therm.\ II} + \sigma_{Koh.\ III} = |\sigma_1 - \sigma_2|$$

und anschließender Differenzbildung

$$\sigma_{Koh.\ III} = |\Delta\sigma - \sigma_{therm.\ II}|$$

oder
ii. als Differenz

$$\sigma_{Koh.\ III} = |\sigma_3 - \sigma_2|.$$

**7.** Verfahren nach vorhergehendem Anspruch, **dadurch gekennzeichnet, dass** die erste Auslagerung des Prüfkörpers durch Lösungsglühen des Prüfkörpers, Abschrecken des Prüfkörpers sowie Wärmebehandlung des Prüfkörpers erfolgt.

**8.** Verfahren nach einem der vorhergehenden Ansprüche, **dadurch gekennzeichnet, dass**

a) das Lösungsglühen des Prüfkörpers bei Temperaturen zwischen 700 und 911 °C, bevorzugt zwischen 750 und 905 °C, insbesondere zwischen 800 und 860 °C und/oder über einen Zeitraum von 30 min bis 24 Stunden, bevorzugt von 30 min bis 5 Stunden, insbesondere von 1 bis 3 Stunden erfolgt,
b) das Abschrecken durch Eintauchen des Prüfkörpers in ein Fluid, insbesondere Wasser erfolgt, und/oder
c) die Wärmebehandlung durch ein oder mehrstufiges Lagern des Prüfkörpers bei Temperaturen zwischen 250 und 750 °C, bevorzugt zwischen 300 und 600 °C, insbesondere zwischen 350 und 550 °C und/oder über einen Zeitraum von bis zu 14 Stunden, bevorzugt von 5 Stunden bis 14 Stunden erfolgt.

**9.** Verfahren nach einem der vorhergehenden Ansprüche, **dadurch gekennzeichnet, dass** die weitere Auslagerung des Prüfkörpers bis zur Ostwald-Reifung durch thermische Überalterung des Prüfkörpers im Mehrphasenbereich erfolgt.

**10.** Verfahren nach dem vorhergehenden Anspruch, **dadurch gekennzeichnet, dass** die thermische Überalterung durch ein oder mehrstufiges Lagern des Prüfkörpers bei Temperaturen zwischen 250 und 750 °C, bevorzugt zwischen 300 und 600 °C, insbesondere zwischen 350 und 550 °C und/oder über einen Zeitraum von 14 Stunden bis 108 Stunden erfolgt.

**11.** Verfahren nach einem der vorhergehenden Ansprüche, **dadurch gekennzeichnet, dass** das Legierungssystem ausgewählt ist aus der Gruppe bestehend aus Fe-Cu-, Fe-Cu-Ni- oder Fe-Cu-Ni-Mn-Legierungen.

**12.** Verfahren nach vorhergehendem Anspruch, **dadurch gekennzeichnet, dass**

a) die Fe-Cu-Legierung einen Cu-Gehalt von 0,1 bis 2 Gew.-%, bevorzugt von 0,3 bis 2 Gew.-%, insbesondere von 0,6 bis 2 Gew.-%,
b) die Fe-Cu-Ni-Legierung einen Cu-Gehalt von 0,1 bis 2 Gew.-%, bevorzugt von 0,3 bis 2 Gew.-%, insbesondere von 0,6 bis 2 Gew.-%; sowie einen Ni-Gehalt von 0,1 bis 10 Gew.-%, bevorzugt von 0,5 bis 5 Gew.-%, insbesondere von 0,8 bis 2 Gew.-%, oder
c) die Fe-Cu-Ni-Mn-Legierung einen Cu-Gehalt von 0,1 bis 2 Gew.-%, bevorzugt von 0,3 bis 2 Gew.-%, insbesondere von 0,6 bis 2 Gew.-%; einen Ni-Gehalt von von 0,1 bis 10 Gew.-%, bevorzugt von 0,5 bis 5 Gew.-%, insbesondere von 0,8 bis 2 Gew.-%; sowie einen Mn-Gehalt von 0,1 bis 8 Gew.-%, bevorzugt von 0,3 bis 5 Gew.-%, insbesondere von 0,5 bis 1,3 Gew.-%

aufweist, wobei sich jeweils der Eisengehalt der jeweiligen Legierung ad 100 Gew.-% addiert.

**13.** Verfahren nach einem der vorhergehenden Ansprüche, **dadurch gekennzeichnet, dass** die an den Prüfkörper angelegte maximale Lastspannung höchstens 50 % der Streckgrenze des Materials des Prüfkörpers beträgt.

**14.** Verfahren nach einem der vorhergehenden Ansprüche, **dadurch gekennzeichnet, dass** die an den Prüfkörper angelegte Lastspannung $\sigma$ von 0,01 bis 100 MPa, bevorzugt von 0,1 bis 50 MPa variiert wird.

**15.** Verfahren nach einem der vorhergehenden Ansprüche, **dadurch gekennzeichnet, dass** die Lastspannung eine Zuglastspannung ist.

## Claims

**1.** Method for the non-destructive quantitative determination of type II internal microstress (type II thermally induced internal stress) of an alloy system containing Fe and Cu, having a Cu content of ≤ 2% by weight, comprising the following steps:

a) determining the load stress value $\sigma_1$ at which the load stress dependency of the maximum Barkhausen noise amplitude $M_{MAX}(\sigma)$ for a test piece made from the alloy system in an initial state has a maximum value,

b) ageing the test piece up to Ostwald ripening,

c) determining the load stress value $\sigma_3$ at which the load stress dependency of the maximum Berkhausen noise amplitude $M_{MAX}(\sigma)$ for a test piece after the ageing up to Ostwald ripening and cooling has a maximum value, as well as

d) determining the type II internal microstress as a difference $\sigma_{therm.\ II} = |\sigma_1 - \sigma_3|$.

2. Method according to the preceding claim, **characterised in that** the ageing of the test piece up to Ostwald ripening occurs by solution annealing the test piece, quenching the test piece as well as thermally over-ageing the test piece.

3. Method according to the preceding claim, **characterised in that** the solution annealing occurs in the solubility range and/or the thermal over-ageing occurs in the multi-phase range.

4. Method according to one of the two preceding claims, **characterised in that**

a) the solution annealing of the test piece occurs at temperatures of between 700 and 911°C, preferably between 750 and 905°C, in particular between 800 and 860°C and/or over a period of time from 10 minutes to 24 hours, preferably from 30 minutes to 5 hours, in particular from 1 to 3 hours,

b) the quenching occurs by submerging the test piece in a fluid, in particular water, and/or

c) the thermal over-ageing occurs by single or multi-stage storage of the test piece at temperatures of between 250 and 750°C, preferably between 300 and 600°C, in particular between 350 and 550°C and/or over a period of time of up to 108 hours, preferably from 5 hours to 108 hours.

5. Method for the non-destructive quantitative determination of type III internal microstress (type III coherency tensile internal stress) of an alloy system containing Fe and Cu, having a Cu content of $\leq 2\%$ by weight, comprising the following steps:

a) first ageing of the test piece up to the exclusive formation of coherent Cu precipitations,

b) determining the load stress value $\sigma_2$ at which the load stress dependency of the maximum value of the Barkhausen noise amplitude $M_{MAX}(\sigma)$ for a test piece after the first ageing and cooling has a maximum value.

c) further ageing of the test piece up to Ostwald ripening,

d) determining the load stress value $\sigma_3$ at which the load stress dependency of the Barkhausen noise amplitude $M_{MAX}(\sigma)$ for a test piece after the further ageing to Ostwald ripening and cooling has a maximum value, as well as

e) determining the type III internal microstress as a difference $\sigma_{Koh.III} = |\sigma_3 - \sigma_2|$.

6. Method according to one of the preceding claims, **characterised in that** it serves for the non-destructive quantitative determination of type III internal microstress (type III. coherency tensile internal stress, $\sigma_{Koh.\ III}$) and type II internal microstress (type II. thermally induced internal stress $\sigma_{therm.\ II}$) and/or the sum of the type II and type III internal microstresses ($\Delta\sigma = \sigma_{therm.\ II} + \sigma_{Koh.\ III}$) of an alloy system containing Fe and Cu, having a Cu content of $\leq 2\%$ by weight and comprises the following steps:

a) determining the load stress value $\sigma_1$ at which the load stress dependency of the maximum Barkhausen noise amplitude $M_{MAX}(\sigma)$ for a test piece made from the alloy system in an initial state has a maximum value,

b) first ageing of the test piece up to the exclusive formation of coherent Cu precipitations,

c) determining the load stress value $\sigma_2$ at which the load stress dependency of the maximum Barkhausen noise amplitude $M_{MAX}(\sigma)$ for a test piece after the first ageing and cooling has a maximum value

d) further ageing of the test piece up to Ostwald ripening,

e) determining of the load stress value $\sigma_3$ at which the load stress dependency of the maximum Barkhausen noise amplitude $M_{MAX}(\sigma)$ for a test piece after the further ageing up to Ostwald ripening and cooling has a maximum value,

f) determining the type II internal microstress ($\sigma_{therm.\ II}$) as a difference $\sigma_{therm.\ II} = |\sigma_1 - \sigma_3|$

g) determining the type III internal microstress by

i. forming the sum of the type II internal microstress and the type III internal microstress as a difference

$$\Delta\sigma = \sigma_{therm.\ II} + \sigma_{Koh.\ III} = |\sigma_1 - \sigma_2|$$

and subsequent difference formation

$$\sigma_{Koh.\ III} = |\ \Delta\sigma - \sigma_{therm.\ II}\ |$$

or
ii. as a difference

$$\sigma_{Koh.\ III} = |\sigma_3 - \sigma_2|.$$

7. Method according to the preceding claim, **characterised in that** the first ageing of the test piece occurs by solution annealing the test piece, quenching the test piece and heat-treating the test piece.

8. Method according to one of the preceding claims, **characterised in that**

> a) the solution annealing of the test piece occurs at temperatures of between 700 and 911°C, preferably between 750 and 905°C, in particular between 800 and 860°C and/or over a period of time from 30 minutes to 24 hours, preferably from 30 minutes to 5 hours, in particular from 1 to 3 hours,
> b) the quenching occurs by submerging the test piece in a fluid, in particular water, and/or
> c) the heat-treatment occurs by single or multi-stage storage of the test piece at temperatures of between 250 and 750°C, preferably between 300 and 600°C, in particular between 350 and 550°C and/or over a period of time up to 14 hours, preferably from 5 hours to 14 hours.

9. Method according to one of the preceding claims, **characterised in that** the further ageing of the test piece up to Ostwald ripening occurs by thermal over-ageing of the test piece in the multi-phase range.

10. Method according to the preceding claim, **characterised in that** the thermal over-ageing occurs by single or multi-stage storage of the test piece at temperatures of between 250 and 750°C, preferably between 300 and 600°C, in particular between 350 and 550°C and/or over a period of time from 14 hours to 108 hours.

11. Method according to one of the preceding claims, **characterised in that** the alloy system is selected from the group consisting of Fe-Cu, Fe-Cu-Ni or Fe-Cu-Ni-Mn alloys.

12. Method according to the preceding claim, **characterised in that**

> a) the Fe-Cu alloy has a Cu-content from 0.1 to 2% by weight, preferably from 0.3 to 2% by weight, in particular from 0.6 to 2% by weight,
> b) the Fe-Cu-Ni alloy has a Cu-content from 0.1 to 2% by weight, preferably from 0.3 to 2% by weight, in particular from 0.6 to 2% by weight; as well as an Ni content from 0.1 to 10% by weight, preferably from 0.5 to 5% by weight, in particular from 0.8 to 2% by weight, or
> c) the Fe-Cu-Ni-Mn alloy has a Cu content from 0.1 to 2% by weight, preferably from 0.3 to 2% by weight, in particular from 0.6 to 2% by weight; an Ni content from 0.1 to 10% by weight, preferably from 0.5 to 5% by weight, in particular from 0.8 to 2% by weight; as well as an Mn content of 0.1 to 8% by weight, preferably from 0.3 to 5% by weight, in particular from 0.5 to 1.3% by weight,

wherein the iron content of the respective alloy adds up to 100% by weight.

13. Method according to one of the preceding claims, **characterised in that** the maximum load stress applied to the test piece amounts to at most 50% of the yield strength of the material of test piece.

14. Method according to one of the preceding claims, **characterised in that** the load stress $\sigma$ applied to the test piece is varied from 0.01 to 100 MPa, preferably from 0.1 to 50 MPa.

15. Method according to one of the preceding claims, **characterised in that** the load stress is a tensile load stress.

**Revendications**

1. Procédé pour la détermination quantitative non destructive de la micro-contrainte interne de type II (contrainte interne thermiquement induite de type II) d'un système d'alliage contenant du Fe et du Cu, ayant une teneur en Cu $\leq 2$ % en poids, comprenant les étapes suivantes :

    a) détermination de la valeur de la contrainte de charge $\sigma_1$, pour laquelle la courbe présentant les variations, en fonction de la contrainte de charge, de l'amplitude maximale du bruit Barkhausen $M_{MAX}(\sigma)$, pour un bloc d'essai constitué du système d'alliage, présente dans un état initial un maximum,
    b) durcissement par précipitation du bloc d'essai jusqu'à la maturation d'Ostwald,
    c) détermination de la valeur de la contrainte de charge $\sigma_3$ pour laquelle la courbe de la variation, en fonction de la contrainte de charge, de l'amplitude maximale du bruit Barkhausen $M_{MAX}(\sigma)$, pour une éprouvette après le durcissement par précipitation jusqu'à la maturation d'Ostwald et refroidissement, présente une valeur maximale, ainsi que
    d) détermination de la micro-contrainte interne de type II en tant que différence $\sigma term.II = |\sigma_1 - \sigma_3|$.

2. Procédé selon la revendication précédente, **caractérisé en ce que** le durcissement par précipitation du bloc d'essai jusqu'à la maturation d'Ostwald est réalisé par recuit de mise en solution du bloc d'essai, trempe du bloc d'essai, ainsi que sur-vieillissement thermique du bloc d'essai.

3. Procédé selon la revendication précédente, **caractérisé en ce que** le recuit de mise en solution a lieu dans le domaine de solubilité, et/ou le sur-vieillissement thermique dans le domaine multiphasique.

4. Procédé selon l'une des deux revendications précédentes, **caractérisé en ce que**

    a) le recuit de mise en solution du bloc d'essai est effectué à des températures comprises entre 700 et 911°C, de préférence entre 750 et 905°C, en particulier entre 800 et 860°C, et/ou sur un laps de temps de 10 min à 24 heures, de préférence de 30 min à 5 heures, en particulier de 1 à 3 heures,
    b) la trempe est réalisée par immersion du bloc d'essai dans un fluide, en particulier l'eau, et/ou
    c) le sur-vieillissement thermique est réalisé par stockage, en une ou plusieurs étapes, du bloc d'essai à des températures comprises entre 250 et 750°C, de préférence entre 300 et 600°C, en particulier entre 350 et 550°C, et/ou sur un laps de temps allant jusqu'à 108 heures, de préférence de 5 heures à 108 heures.

5. Procédé pour la détermination quantitative non destructive de la micro-contrainte interne de type III (contrainte interne de traction en cohérence de type III) d'un système d'alliage contenant du Fe et du Cu, avec une teneur en Cu $\leq 2$ % en poids, comprenant les étapes suivantes :

    a) premier durcissement par précipitation du bloc d'essai jusqu'à formation exclusive de dépôts cohérents de Cu,
    b) détermination de la valeur $\sigma_2$ de la contrainte de charge, pour laquelle la courbe de la variation, en fonction de la contrainte de charge, de la valeur maximale de l'amplitude du bruit Barkhausen $M_{MAX}(\sigma)$, pour une éprouvette, présente après le premier durcissement principal et le refroidissement un maximum,
    c) de nouveau, durcissement par précipitation du bloc d'essai jusqu'à la maturation d'Ostwald,
    d) détermination de la valeur $\sigma_3$ de la contrainte de charge pour laquelle la courbe de la variation, en fonction de la contrainte de charge, de l'amplitude du bruit Barkhausen $M_{MAX}(\sigma)$, pour une éprouvette après le nouveau durcissement par précipitation jusqu'à la maturation d'Ostwald et le refroidissement, présente un maximum, ainsi que
    e) détermination de la micro-contrainte interne de type III en tant que différence $\sigma_{KOH.III} = |\sigma_3 - \sigma_2|$.

6. Procédé selon l'une des revendications précédentes, **caractérisé en ce qu'**il sert à la détermination quantitative non destructive de la micro-contrainte interne de type III (contrainte interne de traction en cohérence de type III, $\sigma_{KOH.III}$) et du type II (contrainte interne thermiquement induite de type II, $\sigma_{therm.II}$) et/ou de la somme des micro-contraintes internes de type II et de type III ($\square\sigma = \sigma_{therm.II} + \sigma_{KOH.III}$) d'un système d'alliage contenant du Fe et du Cu, ayant une teneur en Cu $\leq 2$ % en poids, et comprend les étapes suivantes :

    a) détermination de la valeur $\sigma$ de la contrainte de charge pour laquelle la courbe de la variation, en fonction de la contrainte de charge, de l'amplitude maximale $M_{MAX}(\sigma)$ du bruit Barkhausen, pour un bloc d'essai constitué du système d'alliage dans son état initial, présente un maximum,
    b) premier durcissement par précipitation du bloc d'essai jusqu'à la formation exclusive de dépôts cohérents de Cu,

c) détermination de la valeur $\sigma_2$ de la contrainte de charge pour laquelle la courbe de la variation, en fonction de la contrainte de charge, de l'amplitude maximale $M_{MAX}(\sigma)$ du bruit Barkhausen pour une éprouvette après le premier durcissement par précipitation et le refroidissement, présente un maximum,

d) de nouveau, durcissement par précipitation du bloc d'essai jusqu'à la maturation d'Ostwald,

e) détermination de la valeur $\sigma_3$ de la contrainte de charge pour laquelle la courbe de la variation, en fonction de la contrainte de charge, de l'amplitude maximale $M_{MAX}(\sigma)$ du bruit Barkhausen pour une éprouvette, après le nouveau durcissement par précipitation jusqu'à la maturation d'Ostwald, et le refroidissement, présente un maximum,

f) détermination de la micro-contrainte interne de type II ($\sigma_{therm.II}$) en tant que différence $\sigma_{therm.II} = |\sigma_1 - \sigma_3|$,

g) détermination de la micro-contrainte interne de type III par

    i. formation de la somme de la micro-contrainte interne de type II et de la micro-contrainte interne de type III par la différence

$$\triangle\sigma = \sigma_{therm.II} + \sigma_{KOH\cdot III} = |\sigma_1 - \sigma_2|$$

    puis formation de la différence

$$\sigma_{KON.III} = |\triangle\sigma - \sigma_{thermIII}|,$$

    ou
    ii. par la différence

$$\sigma_{KOH.III} = |\sigma_3 - \sigma_2|.$$

7. Procédé selon la revendication précédente, **caractérisé en ce que** le premier durcissement par précipitation du bloc d'essai est réalisé par recuit de mise en solution du bloc d'essai, trempe du bloc d'essai, ainsi que traitement thermique du bloc d'essai.

8. Procédé selon l'une des revendications précédentes, **caractérisé en ce que**

    a) le recuit de mise en solution du bloc d'essai est effectué à des températures comprises entre 700 et 911°C, de préférence entre 750 et 905°C, en particulier entre 800 et 860°C, et/ou sur un laps de temps de 30 min à 24 heures, de préférence de 30 min à 5 heures, en particulier de 1 à 3 heures,
    b) la trempe est réalisée par immersion du bloc d'essai dans un fluide, en particulier l'eau, et/ou
    c) le traitement thermique est réalisé par stockage en une ou plusieurs étapes du bloc d'essai à des températures comprises entre 250 et 750°C, de préférence entre 300 et 600°C, en particulier entre 350 et 550°C, et/ou sur un laps de temps allant jusqu'à 14 heures, de préférence de 5 heures à 14 heures.

9. Procédé selon l'une des revendications précédentes, **caractérisé en ce que** le nouveau durcissement par précipitation du bloc d'essai jusqu'à la maturation d'Oswald est réalisé par un sur-vieillissement thermique du bloc d'essai dans le domaine multiphasique.

10. Procédé selon la revendication précédente, **caractérisé en ce que** le sur-vieillissement thermique est réalisé par un stockage en une ou plusieurs étapes du bloc d'essai à des températures comprises entre 250 et 750°C, de préférence entre 300 et 600°C, en particulier entre 350 et 550°C, et/ou sur un laps de temps de 14 heures à 108 heures.

11. Procédé selon l'une des revendications précédentes, **caractérisé en ce que** le système d'alliage est choisi dans le groupe consistant en les alliages Fe-Cu, Fe-Cu-Ni ou Fe-Cu-Ni-Mn.

12. Procédé selon la revendication précédente, **caractérisé en ce que**

a) l'alliage Fe-Cu présente une teneur en Cu de 0,1 à 2 % en poids, de préférence de 0,3 à 2 % en poids, en particulier de 0,6 à 2 % en poids,

b) l'alliage Fe-Cu-Ni présente une teneur en Cu de 0,1 à 2 % en poids, de préférence de 0,3 à 2 % en poids, en particulier de 0,6 à 2 % en poids, ainsi qu'une teneur en Ni de 0,1 à 10 % en poids, de préférence de 0,5 à 5 % en poids, en particulier de 0,8 à 2 % en poids, ou

c) l'alliage Fe-Cu-Ni-Mn présente une teneur en Cu de 0,1 à 2 % en poids, de préférence de 0,3 à 2 % en poids, en particulier de 0,6 à 2 % en poids ; une teneur en Ni de 0,1 à 10 % en poids, de préférence de 0,5 à 5 % en poids, en particulier de 0,8 à 2 % en poids ; ainsi qu'une teneur en Mn de 0,1 à 8 % en poids, de préférence de 0,3 à 5 % en poids, en particulier de 0,5 à 1,3 % en poids,

la teneur en fer de chaque alliage assurant le complément à 100 % en poids.

13. Procédé selon l'une des revendications précédentes, **caractérisé en ce que** la contrainte de charge maximale appliquée au bloc d'essai est d'au plus 50 % de la limite élastique du matériau du bloc d'essai.

14. Procédé selon l'une des revendications précédentes, **caractérisé en ce que** la contrainte de charge $\sigma$ appliquée au bloc d'essai varie de 0,01 à 100 MPa, de préférence de 0,1 à 50 MPa.

15. Procédé selon l'une des revendications précédentes, **caractérisé en ce que** la contrainte de charge est une contrainte de charge de traction.

Fig. 1

Fig. 2

$$\Delta\sigma = \sigma_{therm.\,II} + \sigma_{Koh.\,III}$$

Fig. 3

Fig. 4

**Fig. 5**

**Fig. 6**

Fig. 7

IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **I. ALTPETER et al.** Micro-magnetic evaluation of micro residual stresses of the IInd and IIIrd order. *NDT&E International,* 2009, vol. 42, 283-290
- **I. ALTPETER et al.** *NDT&E International,* 2009, vol. 42, 283-290

- **OGI.** Snoek relaxation in a Copper-precipitated alloy steel. *Journal of Alloys and Compounds,* 2000, vol. 310, 432-435